# EUROPÄISCHE PATENTANMELDUNG

(11) **EP 2 955 517 A1**
(43) Veröffentlichungstag der Anmeldung: **16.12.2015**
(21) Anmeldenummer: 14171693.6
(22) Anmeldetag: 10.06.2014
(51) Int. Cl.: G01N 33/50, G01N 1/38

(54) **Verfahren zur Stabilisierung von Körperflüssigkeitsproben durch die Zugabe von Detergenz**

(71) Anmelder: Siemens Healthcare Diagnostics Products GmbH, 35041 Marburg (DE)
(72) Erfinder: Schmidt, Brigitta, 60389 Frankfurt (DE); Muth, Daniel, 35232 Dautphetal (DE)

(57) **Zusammenfassung**

Die vorliegende Erfindung liegt auf dem Gebiet der in vitro-Diagnostik und betrifft ein Verfahren zur Stabilisierung von Körperflüssigkeitsproben, wobei die Körperflüssigkeitsprobe mit einer Detergenz-haltigen Lösung vermischt wird.

## Beschreibung

Die vorliegende Erfindung liegt auf dem Gebiet der in vitro-Diagnostik und betrifft ein Verfahren zur Stabilisierung von Körperflüssigkeitsproben, wobei die Körperflüssigkeitsprobe mit einer Detergenz-haltigen Lösung vermischt wird.

Analytische Testverfahren zur qualitativen oder quantitativen Bestimmung eines Analyten oder einer Aktivität eines Analyten in einer Körperflüssigkeitsprobe sind seit Jahrzehnten bekannt. Weit verbreitet sind Bindungsteste, die auf der Verwendung Analyt-spezifischer Bindungspartner beruhen. Die bekanntesten Bindungsteste sind immunologische Testverfahren, die auf der Verwendung von Analyt-spezifischen Antikörpern beruhen und die sich durch eine hohe Spezifität auszeichnen. Durch die Anwendung von verschiedensten Signalamplifikations- und Detektionstechnologien, die die Messung der Bindungsreaktion von Analyt und Analyt-spezifischem Bindungspartner ermöglichen, wird überdies eine hohe Sensitivität erzielt.

Im klinischen Bereich werden derartige Bindungsteste zunehmend automatisiert mit Hilfe von automatischen Analysesystemen durchgeführt, die eine Vielzahl von Proben sowie eine Vielzahl verschiedenartiger Teste gleichzeitig bearbeiten können. Derartige automatische Analysesysteme sind im Wesentlichen so ausgestaltet, dass sie ein Aliquot aus einem Probenentnahmegefäß entnehmen, in ein Reaktionsgefäß überführen und dann ein oder mehrere Reagenzien aus Reagenzbehältern entnehmen und mit dem Probenaliquot im Reaktionsgefäß vermischen. Der Reaktionsansatz wird inkubiert und anschließend in einer Messeinheit gemessen. Die Auswertung des Messsignals erfolgt automatisch, und dem Anwender wird ein Messsignal bzw. ein Testergebnis angezeigt. Für zahlreiche Testverfahren müssen die zu analysierenden Primärproben, also die unverdünnten Körperflüssigkeitsproben, wie z.B. antikoaguliertes Vollblut, Plasma, Serum, Urin oder ähnliches, vor der Analyse, also bevor sie mit den zur Durchführung notwendigen Reagenzien vermischt werden, verdünnt werden.

Eine Probenverdünnung kann notwendig sein, um mit der gewünschten Messmethode im klinisch relevanten Konzentrationsbereich besser differenzieren zu können. Außerdem hat die Probenverdünnung den Vorteil, dass sogenannte Matrixeffekte minimiert werden, indem die Konzentration probenintrinsischer Störsubstanzen verringert wird.

Üblicherweise werden Primärproben mit gepufferten, isotonischen Lösungen, beispielsweise mit einer phosphatgepufferten Kochsalzlösung, verdünnt. Typische Verdünnungen enthalten 1 Teil Primärprobe und von 9 bis 99 Teile Verdünnungslösung (1:10-Verdünnung bis 1:100-Verdünnung). Je nach zu bestimmendem Analyt und Messmethode können jedoch auch stärkere Verdünnungen möglich oder sogar notwendig sein.

Es wurde beobachtet, dass in mit phosphatgepufferter Kochsalzlösung verdünnten Urin-, Liquor-, Nasensekret- und Ohrensekretproben diverse Analyten, insbesondere Proteine, nach einer gewissen Standzeit nicht mehr zuverlässig nachweisbar sind. In einem konkreten Beispiel wurde festgestellt, dass die Beta-Trace Protein- (BTP-) Konzentration in 1:100-verdünnten Urin-, Liquor-, Nasensekret- und Ohrensekretproben innerhalb von 30 Minuten nach der Verdünnung der Proben signifikant abnimmt, so dass eine zuverlässige quantitative Bestimmung von BTP nicht mehr möglich ist. Dies ist problematisch, da insbesondere bei der Bearbeitung von Proben in einem automatischen Analysesystem häufig keine sofortige Testdurchführung, d.h. keine sofortige Zugabe der erforderlichen Reagenzien zur verdünnten Probe unmittelbar nachdem die Primärprobe verdünnt wurde, möglich ist und eine Stabilität der verdünnten Proben deshalb über die erforderlichen Standzeiten gewährleistet sein muss.

Der vorliegenden Erfindung lag daher die Aufgabe zugrunde, ein Verfahren bereit zu stellen, das die Bereitstellung einer stabilisierten Verdünnung einer Körperflüssigkeitsprobe ermöglicht, so dass eine zuverlässige Analytbestimmung auch noch nach einer gewissen Standzeit der verdünnten Probe gewährleistet ist, bevorzugt nach einer Standzeit von mindestens einer Stunde.

Die Aufgabe wird erfindungsgemäß dadurch gelöst, dass die unverdünnte Körperflüssigkeitsprobe mit einer Detergenz-haltigen Lösung vermischt wird, die mindestens ein nicht-ionisches und/oder mindestens ein ampholytisches Detergenz enthält.

Verdünnungen von Körperflüssigkeitsproben, die durch Zugabe einer Lösung enthaltend mindestens ein nicht-ionisches und/oder mindestens ein ampholytisches Detergenz zu einer unverdünnten Körperflüssigkeitsprobe erhalten werden, sind bei Raumtemperatur noch nach einer Standzeit von drei Stunden stabil, d.h. eine zuverlässige Analytbestimmung ist gewährleistet.

Gegenstand der vorliegenden Erfindung ist also ein Verfahren zur Bereitstellung einer stabilisierten Verdünnung einer Körperflüssigkeitsprobe, wobei die unverdünnte Körperflüssigkeitsprobe mit einer Detergenz-haltigen Lösung vermischt wird, die mindestens ein nicht-ionisches und/oder mindestens ein ampholytisches Detergenz enthält.

Der Begriff "unverdünnte Körperflüssigkeitsprobe" umfasst humane oder tierische Körperflüssigkeiten, die vom Körper ausgeschieden oder abgesondert werden, wie z.B. Urin, Stuhl, Ohrensekret, Nasensekret, Speichel, Ejakulat, Vaginalschleim, Muttermilch oder die dem Körper beispielsweise durch Punktion entnommen werden, wie z.B. Blut, Liquor cerebrospinalis (kurz: Liquor, CSF), Perilymphe (Innenohrflüssigkeit), Fruchtwasser, Synovial- oder Peritonealflüssigkeit. Umfasst sind ferner Körperflüssigkeitsproben wie Plasma oder Serum, die bereits beim Arzt nach oder während der Entnahme einer gängigen, präanalytischen Vorbehandlung, z.B. einer Zentrifugation oder einer Zugabe von Antikoagulanzien (z.B. Citrat, Heparin, EDTA) unterzogen wurden.

Der Begriff "Verdünnung einer Körperflüssigkeitsprobe" umfasst eine Mischung aus einer unverdünnten Körperflüssigkeitsprobe und einer Detergenz-haltigen Lösung, die mindestens ein nicht-ionisches und/oder mindestens ein ampholytisches Detergenz enthält.

In einer Ausführungsform des erfindungsgemäßen Verfahrens sind die Detergenzkonzentration und das Volumen der Detergenz-haltigen Lösung, die mit der unverdünnten Körperflüssigkeitsprobe vermischt wird, so gewählt, dass die Detergenzkonzentration in der erhaltenen Verdünnung der Körperflüssigkeitsprobe 0,001 bis 0,5 Gewichtsprozent, bevorzugt 0,004 bis 0,4 Gewichtsprozent beträgt.

Bevorzugterweise besteht die Detergenz-haltige Lösung aus einer gepufferten Lösung, vorzugsweise einer gepufferten isotonischen Lösung, die mindestens ein nicht-ionisches und/oder mindestens ein ampholytisches Detergenz enthält. Geeignete gepufferte Lösungen sind beispielsweise phosphatgepufferte isotonische Kochsalzlösung (z.B. 0,05 M Natriumdihydrogenphosphat, 0,9 % NaCl, pH-Wert 6,9 bis 7,3), imidazolgepufferte isotonische Kochsalzlösung (z.B. 0,1 M Imidazol, 0,9 % NaCl, pH-Wert 6,9 bis 7,3), glycingepufferte isotonische Kochsalzlösung (z.B. 0,1 M Glycin, 0,9 % NaCl, pH-Wert 8,0 bis 8,2) oder trisgepufferte isotonische Kochsalzlösung (z.B. 0,1 M TRIS, 0,9 % NaCl, pH-Wert 6,9 bis 7,3).

Ein nicht-ionisches Detergenz ist ein Tensid, das keine dissoziierbaren funktionellen Gruppen enthält und sich daher im Wasser nicht in Ionen auftrennt. Wie jedes Tensid sind auch die nicht-ionischen Tenside aus einem unpolaren und einem polaren Teil aufgebaut. Als unpolarer Teil dient meistens ein Fettalkohol (C₁₂-C₁₈) oder Octyl- oder Nonylphenole. Die polaren Gruppen sind hier die Hydroxygruppe und die Ethergruppe. Diese Gruppen sind in Polyethylenglycol oder Monosacchariden enthalten. Vorzugsweise enthält die Detergenz-haltige Lösung mindestens ein nicht-ionisches Detergenz aus der Gruppe Polyoxyethylen(20)-sorbitanmonolaurat (synonym: Polysorbat, Tween® 20), Hydroxypolyethoxydodecan (synonym: Polidocanol, Thesit®), t-Octylphenoxypolyethoxyethanol (synonym: Octoxinol 9, Triton® X-100) und Polyethylenglykol mit einer mittleren relativen Molekülmasse von 6000 (PEG 6000) enthält.

Ein ampholytisches (synonym: amphoteres) Detergenz ist ein Tensid, das sowohl eine negativ als auch eine positiv geladene funktionelle Gruppe besitzt. Wie jedes Tensid sind auch die ampholytischen Tenside aus einem polaren und einem unpolaren Teil aufgebaut. Als unpolarer Teil dient eine Alkylgruppe, als polarer Teil meist eine Carboxylat-Gruppe (R-COO⁻) zusammen mit einer quartären Ammonium-Gruppe (R₄N⁺) Vorzugsweise enthält die Detergenz-haltige Lösung, alternativ zu oder in Kombination mit mindestens einem nicht-ionischen Detergenz- mindestens ein ampholytisches Detergenz aus der Gruppe n-Dodecyl-N,N-dimethyl-3-ammonio-1-propansulfonat (Zwittergent® 3-12), 3-(3-Cholamidopropyl)dimethylammonio]-1-propanesulfonat (CHAPS), 3-(3-Cholamidopropyl)dimethylammonio]-2-hydroxypropan-1-sulfonat (CHAPSO) und Lysolecithine.

Ein weiterer Gegenstand der vorliegenden Erfindung ist ein Verfahren zur Bestimmung eines Analyten in einer Körperflüssigkeitsprobe, wobei eine mit einem erfindungsgemäßen Verfahren bereitgestellte Verdünnung der Körperflüssigkeitsprobe mit einem oder mehreren Reagenzien zum Nachweis des Analyten zu einem Reaktionsansatz vermischt wird, und wobei die Verdünnung der Körperflüssigkeitsprobe mit einem erfindungsgemäßen Verfahren bereitgestellt wurde. Dazu wird die unverdünnte Körperflüssigkeitsprobe zunächst mit einer Detergenz-haltigen Lösung, die mindestens ein nicht-ionisches und/oder mindestens ein ampholytisches Detergenz enthält, vermischt, und die resultierende Verdünnung der Körperflüssigkeitsprobe wird dann mit dem oder den Reagenzien zum Nachweis des Analyten zu einem Reaktionsansatz vermischt.

In einer bevorzugten Ausführungsform umfassen das Reagenz oder die Reagenzien zum Nachweis des Analyten mindestens eine partikuläre Festphase, die mit einem oder mehreren Analyt-spezifischen Bindungspartnern assoziiert ist. Bevorzugte Analyt-spezifische Bindungspartner sind Antigene, Antikörper, Liganden, Rezeptoren, Nukleinsäuren etc.

Vorzugsweise wird zur quantitativen Bestimmung des Analyten die Agglutination der partikulären Festphase bestimmt. Die Agglutination der partikulären Festphase kann zum Beispiel nephelometrisch bestimmt werden. Bindungsteste beruhend auf dem Prinzip der partikelverstärkten Lichtstreuung sind seit etwa 1920 bekannt (zur Übersicht siehe Newman, D.J. et al., Particle enhanced light scattering immunoassay. Ann Clin Biochem 1992; 29: 22-42). Bevorzugterweise werden Polystyrolpartikel mit einem Durchmesser von 0,1 bis 0,5 µm, besonders bevorzugt mit einem Durchmesser von 0,15 0,35 µm verwendet. Weiterhin bevorzugt werden Polystyrolpartikel mit Carboxyl- oder Aldehydfunktionen verwendet.

Alternativ können Nachweisverfahren angewandt werden, bei denen ein erster Analyt-spezifischer Bindungspartner assoziiert mit einer ersten Komponente eines signalbildenden Sytems und ein zweiter Analyt-spezifischer Bindungspartner assoziiert mit einer zweiten Komponente des signalbildenden Sytems verwendet werden und wobei ein messbares Signal, beispielweise Chemilumineszenz, nur entsteht, wenn die beiden Komponenten des signalbildenden Sytems durch gleichzeitige Bindung an ein Analyt-Molekül in räumliche Nähe gelangen und so miteinander wechselwirken können. Unter einer Wechselwirkung zwischen den Komponenten ist insbesondere ein Energietransfer - also die direkte Übertragung von Energie zwischen den Komponenten, z.B. durch Licht- oder Elektronenstrahlung sowie über reaktive chemische Moleküle, wie z. B. kurzlebigen Singulett-Sauerstoff - zu verstehen. Der Energietransfer kann von einer auf eine andere Komponente erfolgen, möglich ist aber auch eine Kaskade verschiedener Substanzen über die der Energietransfer läuft. Zum Beispiel kann es sich bei den Komponenten um ein Paar aus einem Energiespender und einem Energieempfänger handeln, wie beispielsweise Photosensitizer und chemilumineszierendes Agens (EP-A2-0515194, LOCI® Technologie) oder Photosensitizer und Fluorophor (WO 95/06877) oder radioaktives Iod<125> und Fluorophor oder Fluorophor und Fluoreszenz-Quencher (US 3,996,345).

Alternativ können das Reagenz oder die Reagenzien zum Nachweis des Analyten eine nicht-partikuläre Festphase, die mit einem oder mehreren Analyt-spezifischen Bindungspartnern assoziiert ist, umfassen, beispielsweise eine entsprechend beschichtete Mikrotiterplatte oder ein Trägermaterial für die Herstellung von Teststreifen für Point-of-Care-Anwendungen.

Unter einem Analyten ist jede in einer Körperflüssigkeit nachweisbare Substanz zu verstehen, insbesondere Proteine, Proteinkomplexe, Peptide, Hormone, Nukleinsäuren u.s.w.

Ein weiterer Gegenstand der vorliegenden Erfindung ist ein Verfahren zur Bestimmung des Analyten Beta-Trace Protein (BTP; synonym: Prostaglandin H2 D-Isomerase; EC 5.3.99.2) in einer mit dem erfindungsgemäßen Verfahren bereitgestellten Verdünnung einer Körperflüssigkeitsprobe.

Beta-Trace Protein ist ein Hirnprotein, das z.B. nach einem Schädeltrauma oder nach operativen Eingriffen im Nasen- oder Ohrensekret nachgewiesen werden kann und dann auf eine Verletzung des Liquorraums schließen lässt (Reiber, H. et al., Beta-trace protein as sensitive marker for CSF rhinorhea and CSF otorhea. Acta Neurol Scand 2003, 108: 359-362). Im Urin wird Beta-Trace Protein zur Diagnose von Nierenerkrankungen bestimmt.

Es wurde beobachtet, dass eine zuverlässige Bestimmung von Beta-Trace Protein in Verdünnungen von Urin-, Liquor-, Nasensekret- oder Ohrensekretproben nach einer Standzeit der Verdünnung von mehr als 30 Minuten nur dann möglich ist, wenn die Verdünnung mit einer Detergenz-haltigen Lösung, die mindestens ein nicht-ionisches und/oder mindestens ein ampholytisches Detergenz enthält, hergestellt wird.

Eine besonders bevorzugte Ausführungsform betrifft ein Verfahren zur Bestimmung von Beta-Trace Protein in einer erfindungsgemäß bereit gestellten 1:100- bis 1:500-Verdünnung einer Urin-, Liquor-, Nasensekret- oder Ohrensekretprobe, wobei die verdünnte Probe mit mindestens einer partikulären Festphase in Kontakt gebracht wird, die mit einem oder mehreren Bindungspartnern mit Spezifität für Beta-Trace Protein assoziiert ist, beispielsweise mit monoklonalen oder polyklonalen Antikörpern oder Beta-Trace Protein-bindenden Antikörperfragmenten. Die Agglutination der partikulären Festphase wird quantitativ bestimmt, vorzugsweise durch die nephelometrische Bestimmung der partikelverstärkten Lichtstreuung.

Die nachfolgenden Beispiele sind zur Veranschaulichung der Erfindung nicht aber als Einschränkung anzusehen.

### BEISPIELE

### BEISPIEL 1: Immunnephelometrische Bestimmung von Beta-Trace Protein in erfindungsgemäß verdünnten Liquor- und Urinproben

Zur Bestimmung der Beta-Trace Protein-Konzentration in humanen Urin- und Liquorproben wurden die unverdünnten Proben 1:100 (1 Teil Probe + 99 Teile Pufferlösung) mit Imidazolpuffer (0,1 M Imidazol, 0,9 % NaCl, 0,9 g/L Natriumazid, pH-Wert 7,1) oder mit Imidazolpuffer enthaltend 0,00404 oder 0,0404 Gewichtsprozent SDS (Natriumdodecylsulfat) oder -erfindungsgemäß- mit Imidazolpuffer enthaltend 0,00404, 0,0404 oder 0,404 Gewichtsprozent Hydroxypolyethoxydodecan (Thesit®), n-Dodecyl-N,N-dimethyl-3-ammonio-1-propansulfonat (Zwittergent® 3-12), Polyethylenglykol mit einer mittleren relativen Molekülmasse von 6000 (PEG 6000), Polyoxyethylen(20)-sorbitan-monolaurat (Tween® 20) oder t-Octylphenoxypolyethoxyethanol (Triton® X-100) verdünnt. Die erhaltenen Verdünnungen enthielten demnach 0, 0,004, 0,04 bzw. 0,4 Gewichtsprozent Detergenz.

In den verdünnten Proben wurde unmittelbar nach ihrer Herstellung (Ausgangswert) bzw. eine, zwei oder drei Stunden nach ihrer Herstellung und Lagerung bei Raumtemperatur die Beta-Trace Protein-Konzentration immunnephelometrisch bestimmt.

Dazu wurden jeweils 35 µL einer verdünnten Probe mit 150 µL Phosphatpuffer (0,05 M NaHPO₄, 0,9 % NaCl, 1 g/L Natriumazid, pH-Wert 7,1) und mit 50 µL eines ersten Reagenzes enthaltend mit anti-Beta-Trace Protein-Antikörpern beschichtete Latexpartikel und mit 15 µL eines zweiten Reagenzes enthaltend Immunglobulin vom Kaninchen (zur Unterdrückung von Störungen durch Rheumafaktoren) vermischt (N Latex βTP Assay, Siemens Healthcare Diagnostics Products GmbH, Marburg, Deutschland), und die Agglutination der Latexpartikel wurde nephelometrisch bestimmt. Die Beta-Trace Protein-Konzentration wurde durch Auswertung der Messsignale an einer zuvor erstellten Referenzkurve ermittelt.

Die Ergebnisse sind in den Tabellen 1-17 dargestellt.

Messwertabweichungen in Höhe von bis zu 10 % vom Ausgangswert gelten als akzeptabel in Bezug auf die diagnostisch erforderliche Ergebnisrichtigkeit.

**Tabelle 1: Bestimmung der BTP-Konzentration in Urin- und Liquorverdünnungen (1:100) ohne Zusatz von Detergenz (Stand der Technik)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 7536 | 9,67 | --- |
| | 1 | 5342 | 5,67 | - 41,39 % |
| | 2 | 4944 | 5,16 | - 46,69 % |
| | 3 | 4819 | 5,00 | - 48,28 % |
| | | | | |
| **Liquor** | 0 (Ausgangswert) | 4099 | 16,72 | --- |
| | 1 | 2064 | 8,86 | - 47,02 % |
| | 2 | 2061 | 8,85 | - 47,08 % |
| | 3 | 2041 | 8,78 | - 47,52 % |

Aus Tabelle 1 geht hervor, dass Beta-Trace Protein in mit Detergenz-freiem Puffer verdünnten Urin- und Liquorproben mit zunehmender Standzeit der Probenverdünnung nicht mehr zuverlässig nachweisbar ist. Bereits nach einer Stunde Standzeit wurden Konzentrationen gemessen, die von der Konzentration, die unmittelbar nach Verdünnung der Probe ermittelt wurde (Ausgangswert), um über 40 % abweichen.

**Tabelle 2: Bestimmung der BTP-Konzentration in erfindungsgemäßen Urin- und Liquorverdünnungen (1:100) enthaltend 0,4 Gew.-Prozent Hydroxypolyethoxydodecan (Thesit®)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8120 | 11,36 | --- |
| | 1 | 8103 | 11,30 | - 0,48 % |
| | 2 | 8132 | 11,40 | - 0,35 % |
| | 3 | 8239 | 11,76 | 3,53 % |
| | | | | |
| **Liquor** | 0 (Ausgangswert) | 5494 | 23,51 | --- |
| | 1 | 5496 | 23,52 | 0,03 % |
| | 2 | 5521 | 23, 66 | 0, 63 % |
| | 3 | 5381 | 22,88 | - 2,65 % |

Aus Tabelle 2 geht hervor, dass Beta-Trace Protein in mit Detergenz-haltigem Puffer verdünnten Urin- und Liquorproben (0,4 Gew.-Prozent Hydroxypolyethoxydodecan in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 3: Bestimmung der BTP-Konzentration in erfindungsgemäßen Urin- und Liquorverdünnungen (1:100) enthaltend 0,04 Gew.-Prozent Hydroxypolyethoxydodecan (Thesit®)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8218 | 11,68 | --- |
| | 1 | 8111 | 11,33 | - 3,06 % |
| | 2 | 8085 | 11,24 | - 3,77 % |
| | 3 | 8206 | 11,64 | - 0,35 % |
| | | | | |
| **Liquor** | 0 (Ausgangswert) | 5522 | 23,66 | --- |
| | 1 | 5493 | 23,50 | - 0,67 % |
| | 2 | 5535 | 23,73 | 0,30 % |
| | 3 | 5636 | 24,31 | 2,73 % |

Aus Tabelle 3 geht hervor, dass Beta-Trace Protein in mit Detergenz-haltigem Puffer verdünnten Urin- und Liquorproben (0,04 Gew.-Prozent Hydroxypolyethoxydodecan in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 4: Bestimmung der BTP-Konzentration in erfindungsgemäßen Urin- und Liquorverdünnungen (1:100) enthaltend 0,004 Gew.-Prozent Hydroxypolyethoxydodecan (Thesit®)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8215 | 11,67 | --- |
| | 1 | 8079 | 11,22 | - 3,86 % |
| | 2 | 8166 | 11,51 | - 1,40 % |
| | 3 | 8227 | 11,72 | 0,37 % |
| | | | | |
| **Liquor** | 0 (Ausgangswert) | 5414 | 23,07 | --- |
| | 1 | 5398 | 22,98 | - 0,38 % |
| | 2 | 5350 | 22,72 | - 1,50 % |
| | 3 | 5380 | 22,88 | - 0,82 % |

Aus Tabelle 4 geht hervor, dass Beta-Trace Protein in mit Detergenz-haltigem Puffer verdünnten Urin- und Liquorproben mit geringerer Detergenzkonzentration (0,004 Gew.-Prozent Hydroxypolyethoxydodecan in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 5: Bestimmung der BTP-Konzentration in erfindungsgemäßen Urin- und Liquorverdünnungen (1:100) enthaltend 0,4 Gew.-Prozent PEG 600**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8187 | 11,58 | --- |
| | 1 | 8158 | 11,48 | - 0,82 % |
| | 2 | 8035 | 11,08 | - 4,28 % |
| | 3 | 8152 | 11,46 | - 1,00 % |
| | | | | |
| **Liquor** | 0 (Ausgangswert) | 4637 | 19,14 | --- |
| | 1 | 4687 | 19,38 | 1,25 % |
| | 2 | 4655 | 19,22 | 0,44 % |
| | 3 | 4607 | 19,00 | - 0,73 % |

Aus Tabelle 5 geht hervor, dass Beta-Trace Protein in mit Detergenz-haltigem Puffer verdünnten Urin- und Liquorproben (0,4 Gew.-Prozent PEG 6000 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 6: Bestimmung der BTP-Konzentration in erfindungsgemäßen Urin- und Liquorverdünnungen (1:100) enthaltend 0,04 Gew.-Prozent PEG 600**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8129 | 11,39 | --- |
| | 1 | 8103 | 11,30 | - 0,73 % |
| | 2 | 8018 | 11,03 | - 3,14 % |
| | 3 | 8066 | 11,18 | - 1,79 % |
| | | | | |
| **Liquor** | 0 (Ausgangswert) | 4891 | 20,36 | --- |
| | 1 | 4883 | 20,32 | - 0,19 % |
| | 2 | 4863 | 20,22 | - 0,68% |
| | 3 | 4903 | 20,42 | 0,28 % |

Aus Tabelle 6 geht hervor, dass Beta-Trace Protein in mit Detergenz-haltigem Puffer verdünnten Urin- und Liquorproben (0,04 Gew.-Prozent PEG 6000 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 7: Bestimmung der BTP-Konzentration in erfindungsgemäßen Urin- und Liquorverdünnungen (1:100) enthaltend 0,004 Gew.-Prozent PEG 600**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8161 | 11,49 | --- |
| | 1 | 7979 | 10,91 | - 5,06 % |
| | 2 | 7973 | 10,89 | - 5,23 % |
| | 3 | 7764 | 10,28 | - 10,55 % |
| | | | | |
| **Liquor** | 0 (Ausgangswert) | 4867 | 20,24 | --- |
| | 1 | 4701 | 19,44 | - 3,95 % |
| | 2 | 4545 | 18,71 | - 7,55 % |
| | 3 | 4630 | 19,11 | - 5,60 % |

Aus Tabelle 7 geht hervor, dass Beta-Trace Protein in mit Detergenz-haltigem Puffer verdünnten Urin- und Liquorproben (0,004 Gew.-Prozent PEG 6000 in der verdünnten Probe) auch noch nach einer Standzeit der Probenverdünnung von zwei Stunden zuverlässig nachweisbar ist. Die Abweichungen vom Ausgangswert liegen immer unter 10 % und sind damit akzeptabel.

Die über 10 %ige Abweichung vom Ausgangswert in Urinproben nach einer dreistündigen Standzeit verdeutlicht, dass für Urin eine höhere PEG 6000-Konzentration zu bevorzugen ist.

**Tabelle 8: Bestimmung der BTP-Konzentration in erfindungsgemäßen Urin- und Liquorverdünnungen (1:100) enthaltend 0,4 Gew.-Prozent t-Octylphenoxypolyethoxyethanol (Triton® X-100)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8500 | 12,73 | --- |
| | 1 | 8492 | 12,70 | - 0,25 % |
| | 2 | 8456 | 12,56 | - 1,37 % |
| | 3 | 8454 | 12,55 | - 1,43 % |
| | | | | |
| **Liquor** | 0 (Ausgangswert) | 6143 | 27,41 | --- |
| | 1 | 6194 | 27,74 | - 1,21 % |
| | 2 | 6184 | 27,67 | - 0,97 % |
| | 3 | 6218 | 27,90 | - 1,80 % |

Aus Tabelle 8 geht hervor, dass Beta-Trace Protein in mit Detergenz-haltigem Puffer verdünnten Urin- und Liquorproben (0,4 Gew.-Prozent Triton® X-100 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 9: Bestimmung der BTP-Konzentration in erfindungsgemäßen Urin- und Liquorverdünnungen (1:100) enthaltend 0,04 Gew.-Prozent t-Octylphenoxypolyethoxyethanol (Triton® X-100)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8334 | 12,10 | --- |
| | 1 | 8317 | 12,04 | - 0,50 % |
| | 2 | 8228 | 11,72 | - 3,12 % |
| | 3 | 8268 | 11,86 | - 1,96 % |
| | | | | |
| **Liquor** | 0 (Ausgangswert) | 5480 | 23,43 | --- |
| | 1 | 5518 | 23,64 | 0,89 % |
| | 2 | 5484 | 23,45 | 0,10 % |
| | 3 | 5463 | 23,34 | - 0,40 % |

Aus Tabelle 9 geht hervor, dass Beta-Trace Protein in mit Detergenz-haltigem Puffer verdünnten Urin- und Liquorproben (0,04 Gew.-Prozent Triton® X-100 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 10: Bestimmung der BTP-Konzentration in erfindungsgemäßen Urin- und Liquorverdünnungen (1:100) enthaltend 0,004 Gew.-Prozent t-Octylphenoxypolyethoxyethanol (Triton® X-100)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8341 | 12,12 | --- |
| | 1 | 8330 | 12,08 | - 0,33 % |
| | 2 | 8269 | 11,86 | - 2,11 % |
| | 3 | 8291 | 11,94 | - 1,47 % |
| | | | | |
| **Liquor** | 0 (Ausgangswert) | 5011 | 20,96 | --- |
| | 1 | 5092 | 21,37 | 1,97 % |
| | 2 | 4988 | 20,84 | - 0,56 % |
| | 3 | 4935 | 20,58 | - 1,81 % |

Aus Tabelle 10 geht hervor, dass Beta-Trace Protein in mit Detergenz-haltigem Puffer verdünnten Urin- und Liquorproben (0,004 Gew.-Prozent Triton® X-100 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 11: Bestimmung der BTP-Konzentration in erfindungsgemäßen Urin- und Liquorverdünnungen (1:100) enthaltend 0,4 Gew.-Prozent Polyoxyethylen(20)-sorbitan-monolaurat (Tween 20)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8216 | 11,68 | --- |
| | 1 | 8269 | 11,86 | 1,58 % |
| | 2 | 8194 | 11,50 | - 1,50 % |
| | 3 | 8230 | 11,73 | 0,42 % |
| | | | | |
| **Liquor** | 0 (Ausgangswert) | 5568 | 23,92 | --- |
| | 1 | 5616 | 24,19 | 1,14 % |
| | 2 | 5512 | 23,61 | - 1,31 % |
| | 3 | 5583 | 24,01 | 0,36 % |

Aus Tabelle 11 geht hervor, dass Beta-Trace Protein in mit Detergenz-haltigem Puffer verdünnten Urin- und Liquorproben (0,4 Gew.-Prozent Tween® 20 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 12: Bestimmung der BTP-Konzentration in erfindungsgemäßen Urin- und Liquorverdünnungen (1:100) enthaltend 0,04 Gew.-Prozent Polyoxyethylen(20)-sorbitan-monolaurat (Tween® 20)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8340 | 12,12 | --- |
| | 1 | 8240 | 11,76 | - 2,95 % |
| | 2 | 8189 | 11,59 | - 4,40 % |
| | 3 | 8239 | 11,76 | - 2,98 % |
| | | | | |
| **Liquor** | 0 (Ausgangswert) | 5538 | 23,75 | --- |
| | 1 | 5533 | 23,72 | - 0,11 % |
| | 2 | 5388 | 22,92 | - 3,49 % |
| | 3 | 5466 | 23,35 | - 1,67 % |

Aus Tabelle 12 geht hervor, dass Beta-Trace Protein in mit Detergenz-haltigem Puffer verdünnten Urin- und Liquorproben (0,04 Gew.-Prozent Tween® 20 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 13: Bestimmung der BTP-Konzentration in erfindungsgemäßen Urin- und Liquorverdünnungen (1:100) enthaltend 0,004 Gew.-Prozent Polyoxyethylen(20)-sorbitan-monolaurat (Tween® 20)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8254 | 11,81 | --- |
| | 1 | 8178 | 11,55 | - 2,23 % |
| | 2 | 8113 | 11,33 | - 4,05 % |
| | 3 | 8135 | 11,41 | - 3,42 % |
| | | | | |
| **Liquor** | 0 (Ausgangswert) | 5083 | 21,32 | --- |
| | 1 | 5208 | 21,97 | 3,03 % |
| | 2 | 5176 | 21,80 | 2,24 % |
| | 3 | 5158 | 21,70 | 1,80 % |

Aus Tabelle 13 geht hervor, dass Beta-Trace Protein in mit Detergenz-haltigem Puffer verdünnten Urin- und Liquorproben (0,004 Gew.-Prozent Tween® 20 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 14: Bestimmung der BTP-Konzentration in erfindungsgemäßen Urin- und Liquorverdünnungen (1:100) enthaltend 0,4 Gew.-Prozent n-Dodecyl-N,N-dimethyl-3-ammonio-1-propansulfonat (Zwittergent® 3-12)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8342 | 12,13 | --- |
| | 1 | 8209 | 11,66 | - 3,88 % |
| | 2 | 8065 | 11,18 | - 7,81 % |
| | 3 | 8139 | 11,42 | - 5,81 % |
| | | | | |
| **Liquor** | 0 (Ausgangswert) | 5320 | 22,56 | --- |
| | 1 | 5322 | 22,57 | 0,06 % |
| | 2 | 5254 | 22,21 | - 1,55 % |
| | 3 | 5324 | 22,58 | 0,09 % |

Aus Tabelle 14 geht hervor, dass Beta-Trace Protein in mit Detergenz-haltigem Puffer verdünnten Urin- und Liquorproben (0,4 Gew.-Prozent Zwittergent® 3-12 in der verdünnten Probe) auch noch nach einer Standzeit der Probenverdünnung von drei Stunden zuverlässig nachweisbar ist. Die Abweichungen vom Ausgangswert liegen immer unter 10 % und sind damit akzeptabel.

**Tabelle 15: Bestimmung der BTP-Konzentration in erfindungsgemäßen Urin- und Liquorverdünnungen (1:100) enthaltend 0,04 Gew.-Prozent n-Dodecyl-N,N-dimethyl-3-ammonio-1-propansulfonat (Zwittergent® 3-12)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8264 | 11,85 | --- |
| | 1 | 8277 | 11,89 | 0,39 % |
| | 2 | 8174 | 11,54 | - 2,63 % |
| | 3 | 8218 | 11,69 | - 1,36 % |
| | | | | |
| **Liquor** | 0 (Ausgangswert) | 5317 | 22,54 | --- |
| | 1 | 5366 | 22,80 | 1,16 % |
| | 2 | 5201 | 21,93 | - 2,72 % |
| | 3 | 5358 | 22,76 | 0,97 % |

Aus Tabelle 15 geht hervor, dass Beta-Trace Protein in mit Detergenz-haltigem Puffer verdünnten Urin- und Liquorproben (0,04 Gew.-Prozent Zwittergent® 3-12 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

**Tabelle 16: Bestimmung der BTP-Konzentration in erfindungsgemäßen Urin- und Liquorverdünnungen (1:100) enthaltend 0,004 Gew.-Prozent n-Dodecyl-N,N-dimethyl-3-ammonio-1-propansulfonat (Zwittergent® 3-12)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 8149 | 11,45 | --- |
| | 1 | 8304 | 11,99 | 4,68 % |
| | 2 | 8152 | 11,46 | 0,09 % |
| | 3 | 8232 | 11,73 | 2,46 % |
| | | | | |
| **Liquor** | 0 (Ausgangswert) | 5037 | 21,09 | --- |
| | 1 | 4565 | 18,80 | - 10,83 % |
| | 2 | 4580 | 18,87 | - 10,51 % |
| | 3 | 4575 | 18,85 | - 10,61 % |

Aus Tabelle 16 geht hervor, dass Beta-Trace Protein in mit Detergenz-haltigem Puffer verdünnten Urinproben (0,004 Gew.-Prozent Zwittergent® 3-12 in der verdünnten Probe) auch noch nach einer Standzeit der Probenverdünnung von drei Stunden zuverlässig nachweisbar ist. Die Abweichungen vom Ausgangswert liegen immer weit unter 10 % und sind damit akzeptabel.

Die über 10 %ige Abweichung vom Ausgangswert in Liquorproben nach bereits einer einstündigen Standzeit verdeutlicht, dass für Liquor eine höhere Zwittergent® 3-12 -Konzentration zu bevorzugen ist.

**Tabelle 17: Bestimmung der BTP-Konzentration in Urin- und Liquorverdünnungen (1:100) enthaltend 0,004 Gew.-Prozent Natriumdodecylsulfat (SDS)**

| **Probe** | **Standzeit (h)** | **Messsignal (Bit)** | **BTP-Konzentration (mg/L)** | **Abweichung vom Ausgangswert** |
|---|---|---|---|---|
| | | | | |
| **Urin** | 0 (Ausgangswert) | 7836 | 10,48 | --- |
| | 1 | 6617 | 7,67 | - 26,80 % |
| | 2 | 6026 | 6,66 | - 36,44 % |
| | 3 | 5850 | 6,39 | - 39,03 % |
| | | | | |
| **Liquor** | 0 (Ausgangswert) | 4864 | 23,07 | --- |
| | 1 | 3924 | 22,98 | - 21,01 % |
| | 2 | 3516 | 22,72 | - 29,25 % |
| | 3 | 3600 | 22,88 | - 27,58 % |

Aus Tabelle 17 geht hervor, dass Beta-Trace Protein in mit Detergenz-haltigem Puffer verdünnten Urin- und Liquorproben mit einem nicht-erfindungsgemäßen, ionischen Detergenz (0,004 Gew.-Prozent Natriumdodecylsulfat in der verdünnten Probe) mit zunehmender Standzeit der Probenverdünnung nicht mehr zuverlässig nachweisbar ist. Bereits nach einer Stunde Standzeit wurden Konzentrationen gemessen, die von der Konzentration, die unmittelbar nach Verdünnung der Probe ermittelt wurde (Ausgangswert), um fast 30 % abweichen. Die Verwendung von Detergenz-haltigen Lösungen enthaltend ein ionisches Detergenz hat nicht denselben stabilisierenden Effekt wie die erfindungsgemäße Verwendung von Detergenz-haltigen Lösungen enthaltend ein nicht-ionisches oder ein ampholytisches Detergenz.

## Patentansprüche

1. Verfahren zur Bereitstellung einer stabilisierten Verdünnung einer Körperflüssigkeitsprobe, **dadurch gekennzeichnet, dass** die unverdünnte Körperflüssigkeitsprobe mit einer Detergenz-haltigen Lösung vermischt wird, die mindestens ein nicht-ionisches und/oder mindestens ein ampholytisches Detergenz enthält.

2. Verfahren gemäß Anspruch 1, wobei die
Detergenzkonzentration und das Volumen der Detergenz-haltigen Lösung, die mit der unverdünnten Körperflüssigkeitsprobe vermischt wird, so gewählt sind, dass die Detergenzkonzentration in der Verdünnung der Körperflüssigkeitsprobe 0,001 bis 0,5 Gewichtsprozent, bevorzugt 0,004 bis 0,4 Gewichtsprozent beträgt.

3. Verfahren gemäß einem der vorhergehenden Ansprüche, wobei die Detergenz-haltige Lösung ein nicht-ionisches Detergenz aus der Gruppe Polyoxyethylen(20)-sorbitanmonolaurat, Hydroxypolyethoxydodecan, t-Octylphenoxypolyethoxyethanol und Polyethylenglykol mit einer mittleren relativen Molekülmasse von 6000 enthält.

4. Verfahren gemäß einem der vorhergehenden Ansprüche, wobei die Detergenz-haltige Lösung ein ampholytisches Detergenz aus der Gruppe *n*-Dodecyl-N,N-dimethyl-3-ammonio-1-propansulfonat, 3-(3-Cholamidopropyl)dimethylammonio]-1-propanesulfonat, 3-(3-Cholamidopropyl)dimethylammonio]-2-hydroxypropan-1-sulfonat und Lysolecithine enthält.

5. Verfahren gemäß einem der vorhergehenden Ansprüche, wobei die unverdünnte Körperflüssigkeitsprobe ausgewählt ist aus der Gruppe Urin, Liquor, Nasensekret und Ohrensekret.

6. Verfahren zur Bestimmung eines Analyten in einer Körperflüssigkeitsprobe, wobei eine Verdünnung der Körperflüssigkeitsprobe mit einem oder mehreren Reagenzien zum Nachweis des Analyten zu einem Reaktionsansatz vermischt wird, **dadurch gekennzeichnet, dass** die Verdünnung der Körperflüssigkeitsprobe mit einem Verfahren gemäß einem der Ansprüche 1-5 bereitgestellt wurde.

7. Verfahren gemäß Anspruch 6, wobei die Reagenzien zum Nachweis des Analyten eine partikuläre Festphase umfassen, die mit einem oder mehreren Analyt-spezifischen Bindungspartnern assoziiert ist.

8. Verfahren gemäß Anspruch 7, wobei zur quantitativen Bestimmung des Analyten die Agglutination der partikulären Festphase bestimmt wird.

9. Verfahren gemäß Anspruch 8, wobei die Agglutination der
partikulären Festphase nephelometrisch bestimmt wird.

10. Verfahren gemäß einem der Ansprüche 6-9 zur Bestimmung
von Beta-Trace Protein in einer Urin-, Liquor-, Nasensekret- oder Ohrensekretprobe.
